# EUROPEAN PATENT APPLICATION

(11) **EP 4 595 976 A1**
(43) Date of publication of application: **06.08.2025**
(21) Application number: 23873050.1
(22) Date of filing: 26.09.2023
(51) Int. Cl.: A61K 45/00, A61P 35/00

(54) **COMPOSITION FOR TREATING CANCER CONTAINING HISTONE DEACETYLASE INHIBITOR, AND ANTICANCER ADJUVANT FOR CANCER IMMUNOTHERAPY**

(30) Priority: 27.09.2022 KR 20220122474
(71) Applicant: Samsung Life Public Welfare Foundation, Seoul 04348 (KR)
(72) Inventor: CHO, Yong Beom, Seoul 06351 (KR); HONG, Hye Kyung, Seoul 06351 (KR); SHIN, Yong Jae, Seoul 06351 (KR); YU, Ah Ran, Seoul 06351 (KR); CHOI, Soo Min, Seoul 06351 (KR)
(74) Representative: Plougmann Vingtoft a/s
(86) International application number: PCT/KR2023/014721
(87) International publication number: WO 2024/071935

(57) **Abstract**

The present invention relates to: a pharmaceutical composition for treating cancer, the composition containing at least one from the group consisting of a histone deacetylase (hereinafter, referred to as HDAC) 6 inhibitor and a HDAC8 inhibitor; an anticancer adjuvant for cancer immunotherapy; a composition for increasing PD-L1 expression in cancer cells; a cancer treatment method using at least one from the group consisting of a HDAC 6 inhibitor and a HDAC8 inhibitor; a method for increasing PD-L1 expression in cancer cells; a use of at least one from the group consisting of a HDAC 6 inhibitor and a HDAC8 inhibitor for treating cancer; a use thereof as an anticancer adjuvant for cancer immunotherapy; and a use thereof for increasing PD-L1 expression in cancer cells.

## Description

### Technical Field

The present invention was made with the support of the Ministry of Health and Welfare, Republic of Korea, under Sub-project No. HR20C0025020022 within Project Identification No. 1465036429, which was conducted in the research program named "Single-cell multiomics analysis based biomarker and new target development for immune cell therapy" in the research project titled "Research-Driven Hospital fostering R&D" by Samsung Seoul Hospital under management of the Korea Health Industry Development Institute, from 01 January 2022 to 31 December 2022.

The present invention relates to: a pharmaceutical composition for cancer treatment, an anticancer adjuvant for cancer immunotherapy, and a composition for increasing PD-L1 expression in cancer cells, each comprising at least one selected from the group consisting of a histone deacetylase (hereinafter, HDAC) 6 inhibitor and an HDAC8 inhibitor; a cancer treatment method and a method for increasing PD-L1 expression in cancer cells, each using at least one selected from the group consisting of an HDAC 6 inhibitor and an HDAC8 inhibitor; and uses of at least one selected from the group consisting of an HDAC 6 inhibitor and an HDAC8 inhibitor for cancer treatment, for anticancer adjuvant application in cancer immunotherapy, and for increasing PD-L1 expression in cancer cells.

### Background Art

Histone deacetylases (hereinafter, HDACs) are enzymes that regulate the balance between acetylation and deacetylation of histone and non-histone proteins by promoting the hydrolysis of the ε-amide bond of lysine residues, and play an important role in the expression and differentiation of genes and maintenance of cellular homeostasis.

HDACs can bind to gene promoters via corepressors or multi-protein transcriptional complexes, where the enzymes modulate transcription through chromatin modifications without directly binding to DNA.

These HDACs are involved in the regulation of multiple cellular processes, and histone acetyltransferases (HATs) and HDACs affect transcriptional activity by acetylating or deacetylating lysine residues at the N-terminus of histone proteins. Additionally, HDACs are known to regulate the post-translational acetylation of at least 50 non-histone proteins, such as α-tubulin, Hsp90, p53, c-Myc, NF-κB, and E2F.

There are 18 encoded human HDACs, which are classified into Class I (HDAC1, 2, 3, and 8), Class II (HDAC4, 5, 6, 7, 9, and 10), Class III (SIRT1-7), and Class IV (HDAC11).

The overexpression of HDACs in various cancer cells causes the repression of important growth-inhibitory genes to promote cancer cell proliferation. Therefore, HDACs are key drug targets for the development of anticancer drugs and inhibitors thereof have been actively developed.

HDAC inhibitors are generally classified into four types according to the chemical structure: hydroxamic acids, benzamides, cyclic peptides, and short-chain fatty acids. Until now, the U.S. FDA has approved, as anticancer drugs, four HDAC inhibitors, SAHA (vorinostat), FK-228 (romidepsin), PXD101 (belinostat), and LBH589 (panobinostat), and the China National Medical Products Administration has approved HBI-8000 (chidamide) as a therapeutic for T-cell lymphoma.

In addition, about 20 promising HDAC inhibitors are in clinical or pre-clinical stages for various cancers. However, most of the HDAC inhibitors have been observed to cause many side effects, including fatigue, nausea, vomiting, and cardiotoxicity.

New drugs that may be effective in the immunotherapy of colorectal cancer have been developed through many studies, but, unfortunately, most CRC patients belong to the microsatellite stable (MSS) type, which differs from the microsatellite instable (MSI) type characterized by an unstable tumor microenvironment (TME), resulting in poor responsiveness to immunotherapy.

### Disclosure of Invention

### Technical Problem

Therefore, the present inventors investigated whether a specific HDAC isoform can regulate immune-related genes in the tumor microenvironment for colorectal cancer and investigated whether a selective HDAC inhibitor was effective in the treatment of colorectal cancer.

As a result, it was identified that HDAC6 and HDAC8 were important epigenetic regulatory factors of PD-L1 in colorectal cancer, and especially the treatment with HDAC6 and HDAC8 inhibitors further up-regulated PD-L1 expression in microsatellite stable (MSS) colorectal cell lines compared with microsatellite instable (MSI) colorectal cell lines, and thus HDAC6 and HDAC8 inhibitors were effective in the immunotherapy of MSS colorectal cancer patients.

Accordingly, an aspect of the present invention is to provide a pharmaceutical composition for cancer treatment, comprising at least one selected from the group consisting of a histone deacetylase (hereinafter, HDAC) 6 inhibitor and an HDAC8 inhibitor.

Another aspect of the present invention is to provide an anticancer adjuvant for cancer immunotherapy, comprising at least one selected from the group consisting of an HDAC 6 inhibitor and an HDAC8 inhibitor.

Still another aspect of the present invention is to provide a composition for increasing PD-L1 expression in cancer cells, the composition comprising at least one selected from the group consisting of an HDAC 6 inhibitor and an HDAC8 inhibitor.

Still another aspect of the present invention is to provide a cancer treatment method using at least one selected from the group consisting of an HDAC 6 inhibitor and an HDAC8 inhibitor.

Still another aspect of the present invention is to provide a method for increasing PD-L1 expression in cancer cells, the method using at least one selected from the group consisting of an HDAC 6 inhibitor and an HDAC8 inhibitor.

Still another aspect of the present invention is to provide use of at least one selected from the group consisting of an HDAC 6 inhibitor and an HDAC8 inhibitor for cancer treatment.

Still another aspect of the present invention is to provide use of at least one selected from the group consisting of an HDAC 6 inhibitor and an HDAC8 inhibitor for anticancer adjuvant application in cancer immunotherapy.

Still another aspect of the present invention is to provide use of at least one selected from the group consisting of an HDAC 6 inhibitor and an HDAC8 inhibitor for increasing PD-L1 expression in cancer cells.

### Solution to Problem

The present invention relates to: a pharmaceutical composition for cancer treatment, an anticancer adjuvant for cancer immunotherapy, and a composition for increasing PD-L1 expression in cancer cells, each comprising at least one selected from the group consisting of a histone deacetylase (hereinafter, HDAC) 6 inhibitor and an HDAC8 inhibitor; a cancer treatment method and a method for increasing PD-L1 expression in cancer cells, each using at least one selected from the group consisting of an HDAC 6 inhibitor and an HDAC8 inhibitor; and uses of at least one selected from the group consisting of an HDAC 6 inhibitor and an HDAC8 inhibitor for cancer treatment, for anticancer adjuvant application in cancer immunotherapy, and for increasing PD-L1 expression in cancer cells.

Hereinafter, the present invention will be described in more detail.

An embodiment of the present invention is directed to a pharmaceutical composition for cancer treatment, comprising at least one selected from the group consisting of a histone deacetylase (hereinafter, HDAC) 6 inhibitor and an HDAC8 inhibitor.

In the present invention, the HDAC6 inhibitor may be Nexturastat A, but is not limited thereto.

In the present invention, the HDAC8 inhibitor may be PCI-34051, but is not limited thereto.

In the present invention, the pharmaceutical composition for cancer treatment may further comprise an anti-PD-L1 antibody.

As used herein, the term "PD-L1" refers to programmed death-ligand 1 and is also called CD274 or B7-H1. PD-L1 is present on the surface of cells and serves to prevent T cells from attacking, and some tumor cells bear high levels of PD-L1, enabling evasion of the immune system, which is body's natural defense system.

As used herein, the term "anti-PD-L1 antibody" refers to an antibody that can specifically bind to PD-L1.

In the present invention, the anti-PD-L1 antibody may be at least one selected from the group consisting of durvalumab, atezolizumab, avelumab, or any fragment, derivative, conjugate, variant, radiolabeled complex, or biosimilar thereof, but is not limited thereto.

In the present invention, the cancer may be at least one selected from the group consisting of colorectal cancer, pancreatic cancer, gastric cancer, and lung cancer.

In the present invention, the cell line targeted by the pharmaceutical composition for cancer treatment may be an MSS cell line, and examples thereof may be SW620, SW480, LS513, HT29, LS1034, or CT26.

In the present invention, the pharmaceutical composition for cancer treatment may further contain at least one appropriate additive selected from the group consisting of a carrier, an excipient, a disintegrant, a sweetener, a coating agent, a leavening agent, a lubricant, a glidant, a flavoring agent, an antioxidant, a buffer, a bacteriostat, a diluent, a dispersant, a surfactant, a binder, and a lubricant, which are commonly used to prepare the pharmaceutical composition.

In the present invention, the pharmaceutical composition for cancer treatment may be administered to a subject in a common manner via an intravenous, intra-arterial, intraperitoneal, intramuscular, intrasternal, transdermal, intranasal, inhalational, topical, rectal, oral, intraocular, or intradermal route.

In the present invention, the dose of the pharmaceutical composition for cancer treatment may vary depending on the condition and body weight of a subject, type and severity of disease, the formulation of a drug, and the route and period of administration, and may be appropriately selected by a person skilled in the art. For example, the daily dose may be 0.01 to 200 mg/kg, 0.1 to 200 mg/kg, 0.1 to 100 mg/kg, or 0.1 to 50 mg/kg. The administration may be carried out once daily or divided into several doses, and the range of the present invention is not limited thereto.

In the present invention, the subject may be a mammal, for example, a human or a mouse, but is not limited thereto.

Another embodiment of the present invention is directed to an anticancer adjuvant for cancer immunotherapy, comprising at least one selected from the group consisting of an HDAC 6 inhibitor and an HDAC8 inhibitor.

In the present invention, the HDAC6 inhibitor may be Nexturastat A, but is not limited thereto.

In the present invention, the HDAC8 inhibitor may be PCI-34051, but is not limited thereto.

In the present invention, the anticancer adjuvant for cancer immunotherapy may further contain an anti-PD-L1 antibody.

In the present invention, the cancer may be at least one selected from the group consisting of colorectal cancer, pancreatic cancer, gastric cancer, and lung cancer.

In the present invention, the cell line targeted by the anticancer adjuvant for cancer immunotherapy may be an MSS cell line, and examples thereof may be SW620, SW480, LS513, HT29, LS1034, or CT26.

In the present invention, the anticancer adjuvant for cancer immunotherapy may further contain at least one appropriate additive selected from the group consisting of a carrier, an excipient, a disintegrant, a sweetener, a coating agent, a leavening agent, a lubricant, a glidant, a flavoring agent, an antioxidant, a buffer, a bacteriostat, a diluent, a dispersant, a surfactant, a binder, and a lubricant, which are commonly used to prepare the pharmaceutical composition.

In the present invention, the anticancer adjuvant for cancer immunotherapy may be administered to a subject in a common manner via an intravenous, intra-arterial, intraperitoneal, intramuscular, intrasternal, transdermal, intranasal, inhalational, topical, rectal, oral, intraocular, or intradermal route.

In the present invention, the dose of the anticancer adjuvant for cancer immunotherapy may vary depending on the condition and body weight of a subject, type and severity of disease, the formulation of a drug, and the route and period of administration and may be appropriately selected by a person skilled in the art. For example, the daily dose may be 0.01 to 200 mg/kg, 0.1 to 200 mg/kg, 0.1 to 100 mg/kg, or 0.1 to 50 mg/kg. The administration may be carried out once daily or divided into several doses, and the range of the present invention is not limited thereto.

In the present invention, the subject may be a mammal, for example, a human or a mouse, but is not limited thereto.

Still another embodiment of the present invention is directed to a composition for increasing PD-L1 expression in cancer cells, the composition comprising at least one selected from the group consisting of an HDAC 6 inhibitor and an HDAC8 inhibitor.

In the present invention, the HDAC6 inhibitor may be Nexturastat A, but is not limited thereto.

In the present invention, the HDAC8 inhibitor may be PCI-34051, but is not limited thereto.

In the present invention, the cancer may be at least one selected from the group consisting of colorectal cancer, pancreatic cancer, gastric cancer, and lung cancer.

In the present invention, the cell line targeted by the anticancer adjuvant for cancer immunotherapy may be an MSS cell line, and examples thereof may be SW620, SW480, LS513, HT29, LS1034, or CT26.

Still another embodiment of the present invention is directed to a cancer treatment method comprising administering to a subject at least one selected from the group consisting of an HDAC 6 inhibitor and an HDAC8 inhibitor.

In the present invention, the HDAC6 inhibitor may be Nexturastat A, but is not limited thereto.

In the present invention, the HDAC8 inhibitor may be PCI-34051, but is not limited thereto.

In the present invention, the cancer treatment method may be implemented by administering to a subject the anti-PD-L1 antibody simultaneously or sequentially with at least one selected from the group consisting of an HDAC 6 inhibitor and an HDAC8 inhibitor.

In the present invention, the cancer may be at least one selected from the group consisting of colorectal cancer, pancreatic cancer, gastric cancer, and lung cancer.

In the present invention, the cell line targeted by the cancer treatment method may be an MSS cell line, and examples thereof may be SW620, SW480, LS513, HT29, LS1034, or CT26.

In the present invention, the subject may be a mammal, for example, a human or a mouse, but is not limited thereto.

In the present invention, the administration may be conducted via an intravenous, intra-arterial, intraperitoneal, intramuscular, intrasternal, transdermal, intranasal, inhalational, topical, rectal, oral, intraocular, or intradermal route.

In the present invention, the dose may vary depending on the condition and body weight of a subject, type and severity of disease, the formulation of a drug, and the route and period of administration and may be appropriately selected by a person skilled in the art. For example, the daily dose may be 0.01 to 200 mg/kg, 0.1 to 200 mg/kg, 0.1 to 100 mg/kg, or 0.1 to 50 mg/kg. The administration may be carried out once daily or divided into several doses, and the range of the present invention is not limited thereto.

In the present invention, the cancer treatment method may be implemented by administering to a subject at least one selected from the group consisting of an HDAC 6 inhibitor and an HDAC8 inhibitor at intervals of 1 to 3 days, or 1 to 2 days, for example, at 1-day intervals, simultaneously with the anti-PD-L1 antibody at intervals of 2 to 4 days, or 2 to 3 days, for example, at 3-day intervals, but is not limited thereto.

In the present invention, the cancer treatment method may be implemented by administering to a subject at least one selected from the group consisting of an HDAC 6 inhibitor and an HDAC8 inhibitor at 0.01 to 200 mg/kg, 0.1 to 200 mg/kg, 0.1 to 100 mg/kg, or 0.1 to 50 mg/kg, for example, 20 mg/kg, per dose, and administering the anti-PD-L1 antibody at 0.01 to 200 mg/kg, 0.1 to 200 mg/kg, 0.1 to 100 mg/kg, 0.1 to 50 mg/kg, or 0.1 to 20 mg/kg, per dose, for example, 10 mg/kg, but is not limited thereto.

Still another embodiment of the present invention is directed to a method for increasing PD-L1 expression in cancer cells, the method using at least one selected from the group consisting of an HDAC 6 inhibitor and an HDAC8 inhibitor.

In the present invention, the HDAC6 inhibitor may be Nexturastat A, but is not limited thereto.

In the present invention, the HDAC8 inhibitor may be PCI-34051, but is not limited thereto.

In the present invention, the cancer may be at least one selected from the group consisting of colorectal cancer, pancreatic cancer, gastric cancer, and lung cancer.

In the present invention, the cell line targeted by the anticancer adjuvant for cancer immunotherapy may be an MSS cell line, and examples thereof may be SW620, SW480, LS513, HT29, LS1034, or CT26.

Still another embodiment of the present invention is directed to use of at least one selected from the group consisting of an HDAC6 inhibitor and an HDAC8 inhibitor for cancer treatment.

Still another embodiment of the present invention is directed to use of at least one selected from the group consisting of an HDAC 6 inhibitor and an HDAC8 inhibitor for anticancer adjuvant application in cancer immunotherapy.

Still another aspect of the present invention is directed to use of at least one selected from the group consisting of an HDAC 6 inhibitor and an HDAC8 inhibitor for increasing PD-L1 expression in cancer cells.

### Advantageous Effects of Invention

The present invention relates to: a pharmaceutical composition for cancer treatment, an anticancer adjuvant for cancer immunotherapy, and a composition for increasing PD-L1 expression in cancer cells, each comprising at least one selected from the group consisting of a histone deacetylase (hereinafter, HDAC) 6 inhibitor and an HDAC8 inhibitor; a cancer treatment method and a method for increasing PD-L1 expression in cancer cells, each using at least one selected from the group consisting of an HDAC 6 inhibitor and an HDAC8 inhibitor; and uses of at least one selected from the group consisting of an HDAC 6 inhibitor and an HDAC8 inhibitor for cancer treatment, for anticancer adjuvant application in cancer immunotherapy, and for increasing PD-L1 expression in cancer cells.

### Brief Description of Drawings

FIG. 1 shows the results of measuring RNA and protein levels of PD-L1 upon treatment with HDAC3 siRNA according to an example of the present invention.
FIG. 2 shows the results of measuring RNA and protein levels of PD-L1 upon treatment with an HDAC3 inhibitor according to an example of the present invention.
FIG. 3 shows the results of measuring RNA and protein levels of PD-L1 upon treatment with HDAC6 siRNA according to an example of the present invention.
FIG. 4 shows the results of measuring RNA and protein levels of PD-L1 upon treatment with an HDAC6 inhibitor according to an example of the present invention.
FIG. 5 shows the results of measuring RNA and protein levels of PD-L1 upon treatment with HDAC8 siRNA according to an example of the present invention.
FIG. 6 shows the results of measuring RNA and protein levels of PD-L1 upon treatment with an HDAC8 inhibitor according to an example of the present invention.
FIGS. 7A and 7B show the results of examining siRNA efficacy by determining whether the expression levels of HDAC6 and HDAC8 were reduced upon treatment with HDAC6 siRNA and HDAC8 siRNA, respectively, according to an example of the present invention.
FIGS. 7C and 7D show the results of measuring expression levels of PD-L1 upon treatment with HDAC6 siRNA and HDAC8 siRNA, according to an example of the present invention.
FIGS. 8A to 8C show the results of measuring expression levels of PD-L1 upon treatment with an HDAC6 inhibitor and an HDAC8 inhibitor at concentrations of ½ IC50 and ¼ IC50 according to an example of the present invention.
FIG. 9A schematically shows HDAC6 inhibitor treatment conditions according to an example of the present invention.
FIGS. 9B and 9C show the results of measuring the volume and weight of cancer cells upon treatment with an HDAC6 inhibitor in an animal test according to an example of the present invention.
FIG. 9D shows the results of measuring the body weight of mice upon treatment with an HDAC6 inhibitor in an animal test according to an example of the present invention.
FIG. 9E shows the results of observing dead cells upon treatment with an HDAC6 inhibitor in an animal test according to an example of the present invention.
FIGS. 10A to 10C show the results of changes of CD3+ T cells, CD4+ T cells, CD8+ T cells, regulatory T cells, M1 macrophages, and M2 macrophages upon treatment with an HDAC6 inhibitor in an animal experiment according to an example of the present invention.
FIG. 11 shows the results of expression level changes of PD-L1, IRF-1, TNF-a, IFN-γ, and IL-6 upon treatment with an HDAC6 inhibitor in an animal experiment according to an example of the present invention.
FIGS. 12A to 12C show the results of validating the hypothesis that an HDAC6 inhibitor suppresses CT26 tumor growth in a CD8 T cell-dependent manner according to an example of the present invention.
FIGS. 13A and 13B show the results of measuring expression levels PD-L1 and other immune-related genes and volume change of cancer cells upon treatment with an HDAC6 inhibitor and an HDAC8 inhibitor in combination in an animal experiment according to an example of the present invention.
FIG. 14 shows the results of measuring basal PD-L1 expression levels in MSI and MSS colorectal cell lines.
FIG. 15 shows the results of measuring PD-L1 and B7H3 expression levels by the treatment with an HDAC6 inhibitor in MSI and MSS colorectal cell lines.

### Best Mode for Carrying out the Invention

The present invention is directed to a pharmaceutical composition for cancer treatment, comprising at least one selected from the group consisting of a histone deacetylase (hereinafter, HDAC) 6 inhibitor and an HDAC8 inhibitor.

### Mode for Carrying out the Invention

Hereinafter, the present invention will be described in more detail by the following exemplary embodiments. However, these exemplary embodiments are used only for illustration, and the scope of the present invention is not limited by these exemplary embodiments.

### Experimental Example 1. Analysis of expression patterns of HDAC isozymes and immune-related genes using TCGA data

The expression levels of HDAC isozymes and immune-related genes were analyzed using TCGA data. The results are shown in Table 1 below, and especially, the correlations between HDAC6 and immune-related genes are shown in Table 2 below.

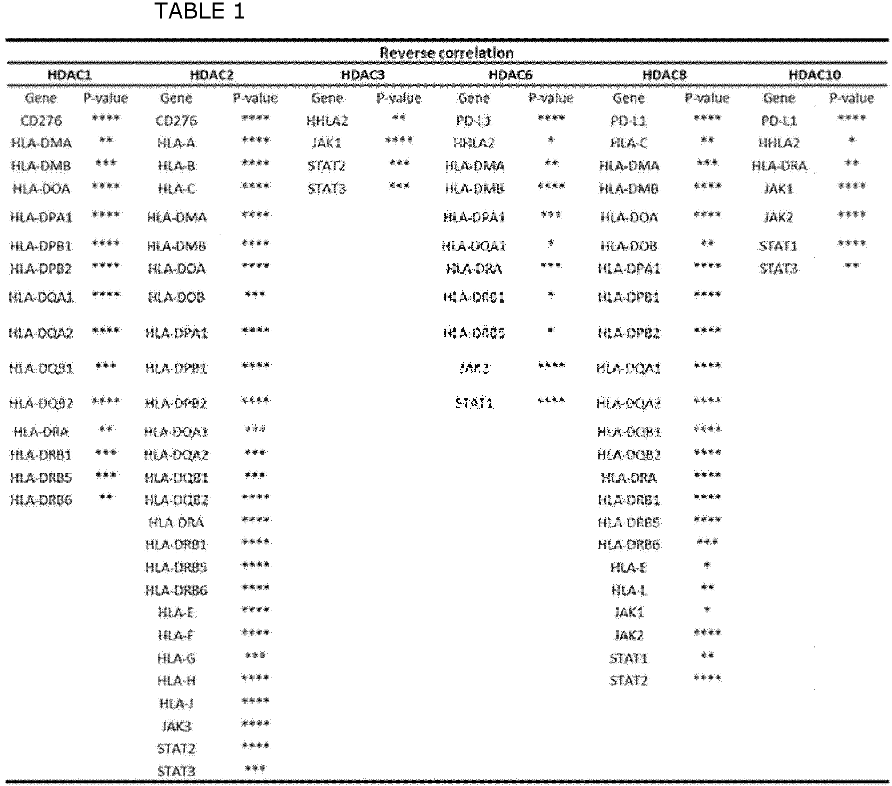

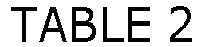

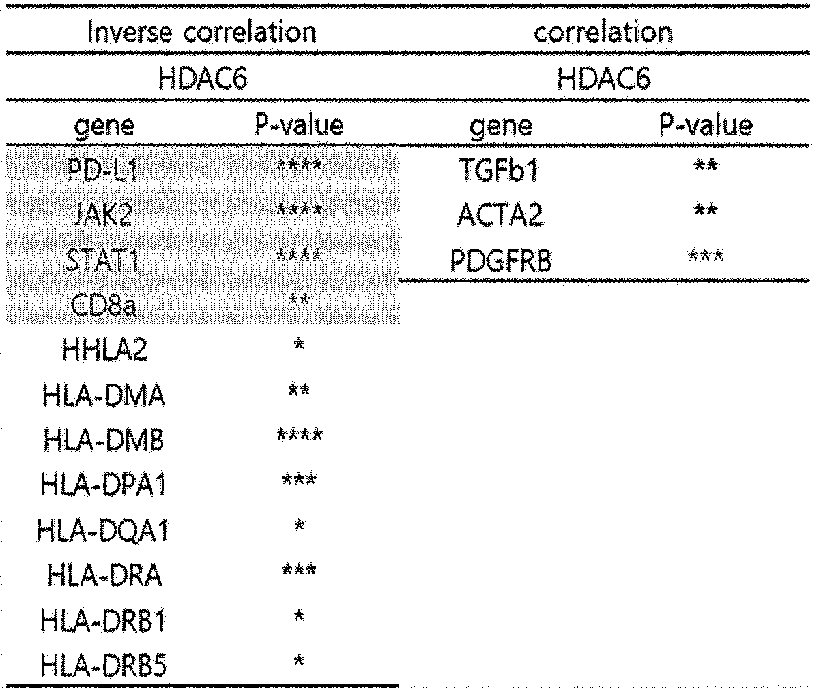

As can be confirmed in Table 1, HDAC6, HDAC8, and HDAC10 had negative correlations with PD-L1. The HDACs also exhibited negative correlations with immune-related genes other than PD-L1. Particularly, as can be confirmed in Table 2, HDAC6 exhibited negative correlations with PD-L1, JAK2, STAT1, and CD8a.

### Experimental Example 2. Measurement of IC50 concentration

The IC₅₀ concentration, which is the concentration at which 50% of cells are dead, was obtained by treating each cell line with an inhibitor at different concentrations for 48 hours and then determining the concentrations at which 50% of the cells were dead. The results are shown in Table 3 below. Especially, the results obtained by treating various MSS cell lines (SW620, LS513, HT29, and CT26) with HDAC6i are shown in Table 4 below.

**TABLE 3**

| | Group | Cell line | IC50(mean±SD) |
|---|---|---|---|
| HDAC3i (RGFP966) | MSS | SW620 | 23.78(±2.84) |
| | | LS513 | 43.05(±2.88) |
| | MSI | DLD-1 | 118.96(±8.74) |
| HDAC6i (Nexturatstat A) | MSS | SW620 | 6.8(±0.52) |
| | | LS513 | 26.9(±2.72) |
| | MSI | DLD-1 | 10.83(±0.64) |
| HDAC8i (PCl-34051) | MSS | SW620 | 31.79(±1.03) |
| | | LS513 | 19.56(±2.88) |
| | MSI | DLD-1 | 6.36(±1.61) |

**TABLE 4**

| (uM) | | |
|---|---|---|
| Nexturastat A(HDAC6i) | | |
| Group | Cell line | IC₅₀(mean±SD) |
| MSS (48hr) | SW620 | 6.8(±0.52) |
| | LS513 | 26.9(±2.72) |
| | HT29 | 11.3(±0.75) |
| | CT26 | 27.08(±0.25) |

### Experimental Example 3. Measurement of PD-L1 expression by HDAC3 expression

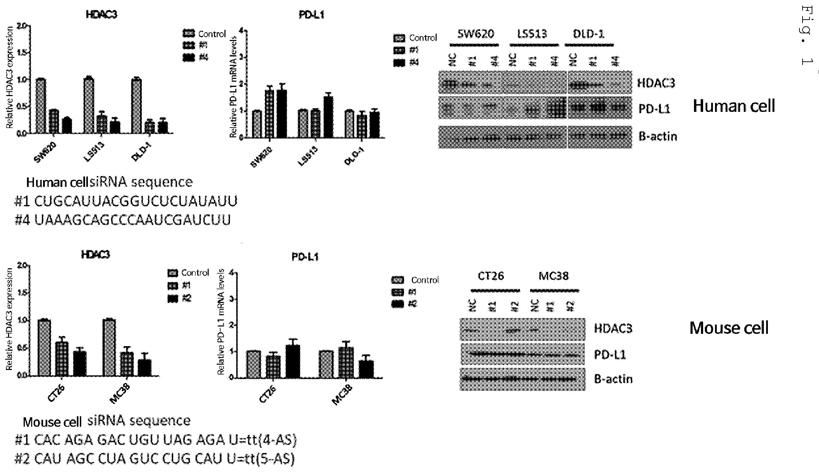

The HDAC3 knockdown experiment was conducted using Lipofectamine RNAiMAX (Invitrogen) through reverse transfection. Cells were harvested after 48 hours, and RNA and proteins were obtained and used for the experiment. A serum-free medium (Opti-MEM; Gibco, 31985070) was used, and the Lipofectamine RNAiMAX and 100 nM siRNA were mixed at a ratio of 1:1 and incubated at room temperature for 30 minutes. Especially, the siRNA was diluted in serum-free medium. After 30 minutes, the Opti-MEM-siRNA mixture was incubated with cells at the following cell numbers: SW620: 8X10⁵, LS513: 1X10⁶, DLD-1: 5X10⁵, CT26: 2X10⁵, and MC38: 1X10⁵), and after 24 hours, the medium was exchanged with fresh medium, after which the cells were incubated for 48 hours. The results are shown in FIG. 1.

As can be confirmed from FIG. 1, the reduction in HDAC3 expression resulted in small increases in PD-L1 in the MSS cell lines (SW620, LS513, and CT26) and, in some cases, even resulted in a decrease in the mouse cells.

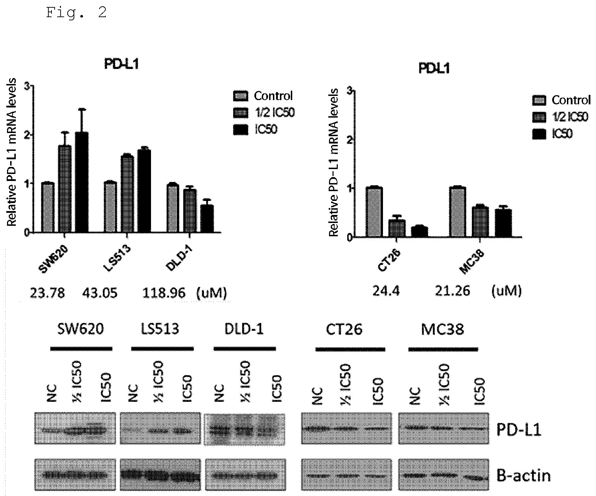

The HDAC3 inhibitor experiment was conducted by harvesting cells 48 hours after the treatment at each inhibitor concentration and obtaining RNA and proteins. RGFP966 (S7229) purchased from SellekChem was used as an HDAC3 inhibitor. Specifically, SW620, LS513, DLD-1, CT26, and MC38 were individually cultured, and the experiments were conducted using the IC₅₀ and 1/2 IC₅₀ concentration on the basis of the previously determined IC₅₀ concentration. RNA and protein samples were collected 48 hours after RGFP966 treatment. The RNA samples were isolated using the TRIzol, isopropanol, and chloroform, and the protein samples were collected by lysis with a lysis buffer (Roche, 4719956001). RNA was synthesized into cDNA using a cDNA synthesis kit (Bioneer, K-2044-B), and real-time PCR was performed using a method according to SYBR (Applied Biosystems, 4367659). Proteins were quantified using the Bradford assay (Bio-Rad, 50000006), and subjected to loading, transfer, blocking (3% skim milk; BD, 232100), primary antibody (24 h, 4°C), and secondary antibody sequentially, thereby developing PD-L1. The results are shown in FIG. 2.

As can be confirmed from FIG. 2, the use of HDAC3 as an inhibitor resulted in small increases (compared with HDAC6 and HDAC8) in PD-L1 expression in the MSS cell lines (SW620, LS513, and CT26) and showed a decrease tendency in the mouse cells.

### Experimental Example 4. Measurement of PD-L1 expression by HDAC6 expression

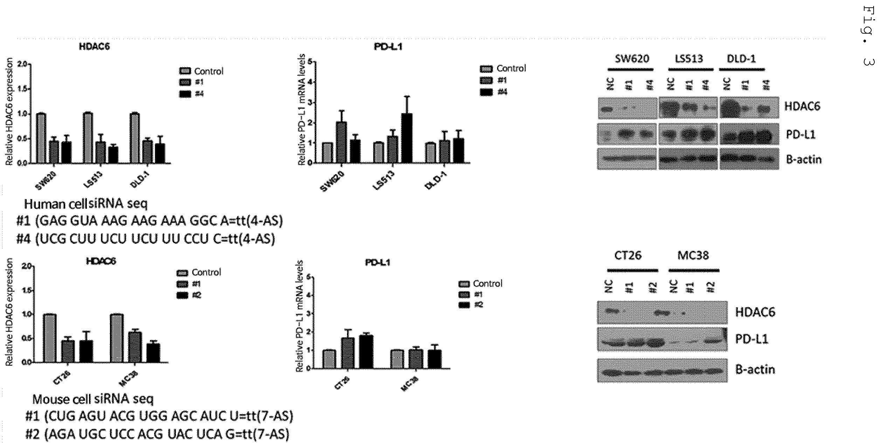

The HDAC6 knockdown experiment was conducted by the same method as in the HDAC3 knockdown experiment of Experimental Example 3, and the results are shown in FIG. 3.

As can be confirmed from FIG. 3, the reduction in HDAC6 expression resulted in increases in PD-L1 expression at the RNA and protein levels in the MSS cell lines (SW620, LS513, and CT26) but no significant change in PD-L1 expression in the MSI cell lines (DLD-1 and MC38).

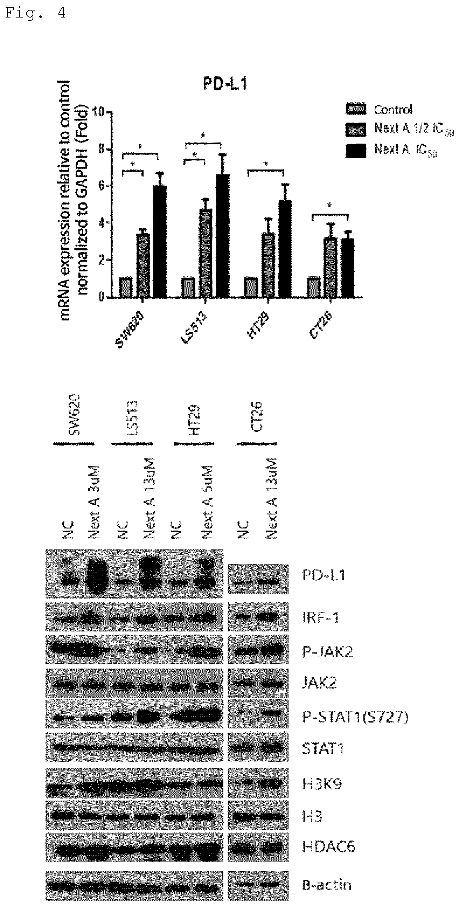

The HDAC6 inhibitor experiment was conducted by the same method as in the HDAC3 inhibitor experiment of Experimental Example 3, and Nexturastat A HDAC6 selective inhibitor purchased from SellekChem was used as an HDAC6 inhibitor. The results are shown in FIG. 4.

As can be confirmed from FIG. 4, inhibition of HDAC6 by an inhibitor led to increase in PD-L1 expression at the RNA and protein levels in the MSS cell lines (SW620, LS513, HT29, and CT26) depending on the concentration.

### Experimental Example 5. Measurement of PD-L1 expression by HDAC8 expression

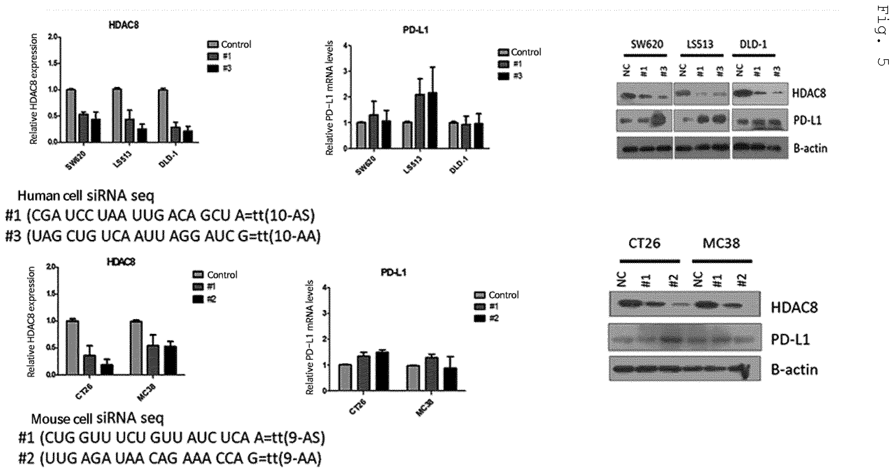

The HDAC8 knockdown experiment was conducted by the same method as in the HDAC3 knockdown experiment of Experimental Example 3, and the results are shown in FIG. 5.

As can be confirmed from FIG. 5, the reduction in HDAC8 expression resulted in increases in PD-L1 expression at the RNA and protein levels in the MSS cell lines (SW620, LS513, and CT26).

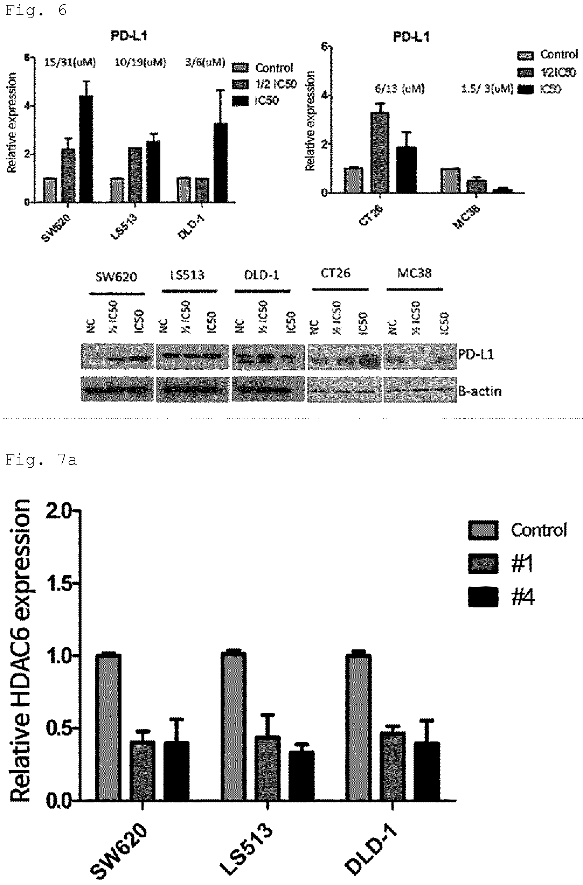

The HDAC8 inhibitor experiment was conducted by the same method as in the HDAC3 inhibitor experiment of Experimental Example 3, and PCI-34051 HDAC8 selective inhibitor purchased from SellekChem was used as an HDAC8 inhibitor. The results are shown in FIG. 6.

As can be confirmed from FIG. 6, inhibition of HDAC8 by an inhibitor led to increase in PD-L1 expression at the RNA and protein levels in the MSS cell lines (SW620, LS513, and CT26) depending on the concentration.

### Experimental Example 6. Measurement of PD-L1 expression by HDAC6 and HDAC8 expression

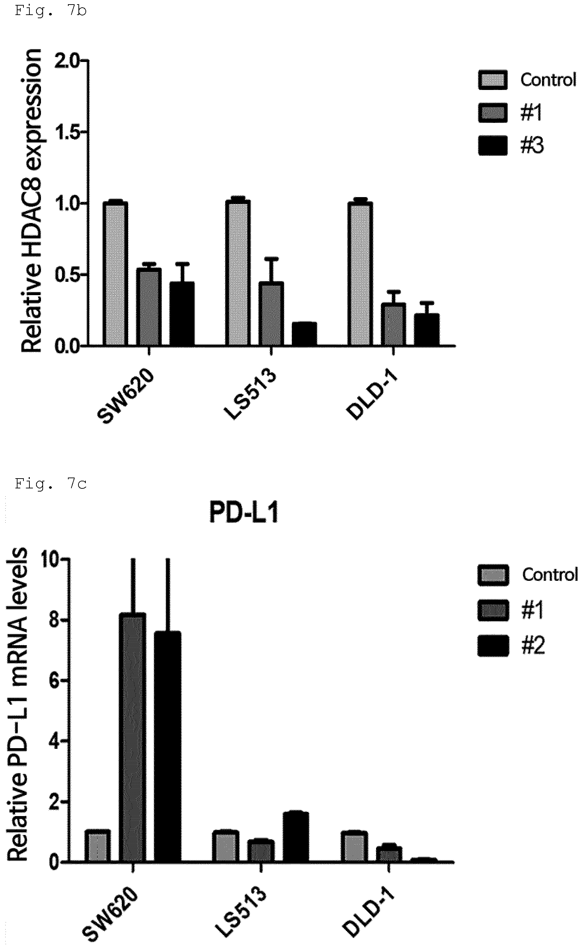

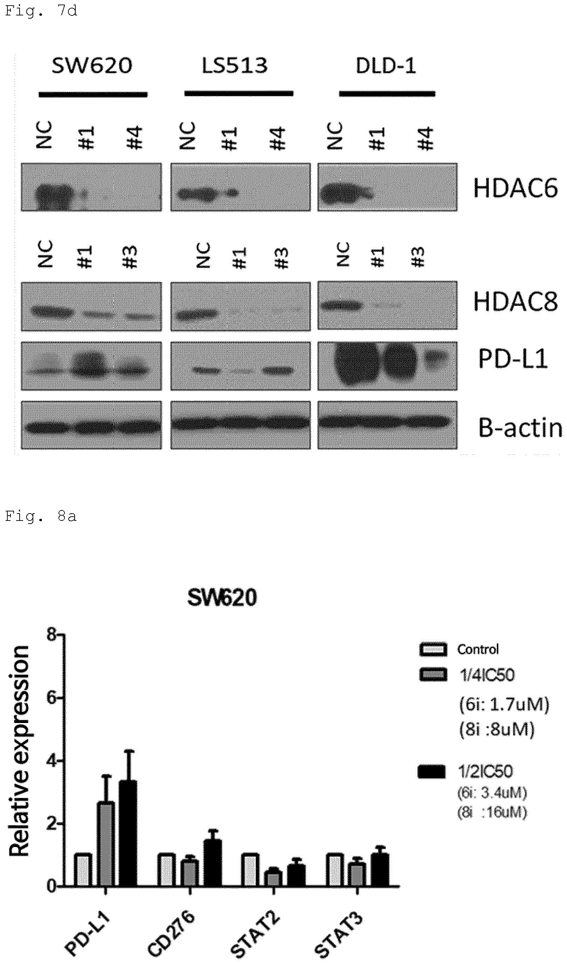

The HDAC6 and HDAC8 knockdown experiment was conducted by the same method as in the HDAC3 knockdown experiment of Experimental Example 3, and the results are shown in FIGS. 7A to 7D.

The same siRNA sequences as used for the individual knockdown experiments were used. In FIG. 7C, #1 indicates 6 siRNA #1 + 8 siRNA #1, and #2 indicates 6 siRNA #4 + 8 siRNA #3.

As can be confirmed from FIGS. 7A to 7D, HDAC6+8i showed an increase tendency in PD-L1 expression in the MSS cell lines (SW620 and LS513).

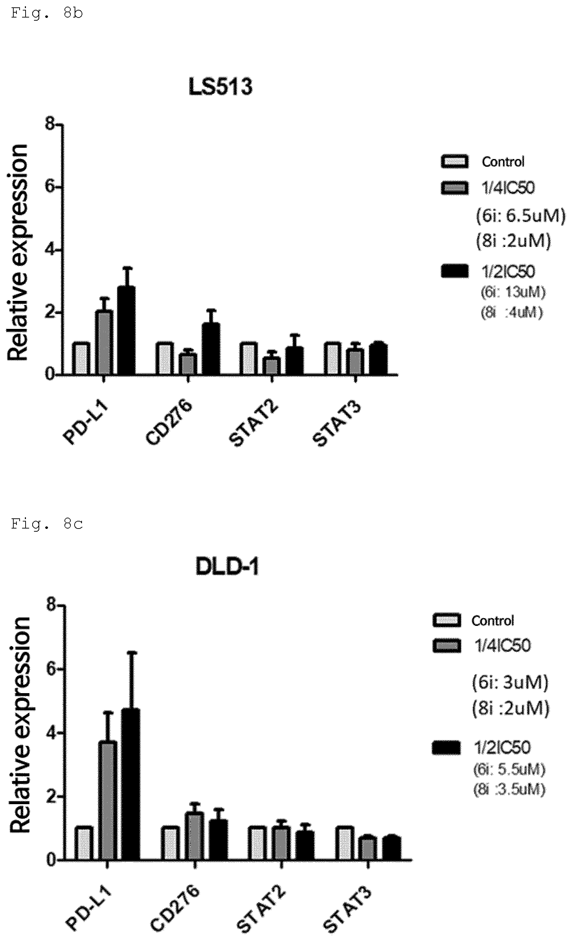

The HDAC6 and HDAC8 inhibitor experiment was conducted by harvesting cells 48 hours after the treatment and then obtaining RNA and proteins. The results are shown in FIGS. 8A to 8C.

As can be confirmed from FIGS. 8A to 8C, the treatment with the HDAC6 and HDAC8 inhibitor at the concentrations of ½ IC50, and ¼ IC50 showed increase tendencies in PD-L1 expression in all of the human colorectal cell lines (SW620, LS513, and DLD-1).

### Experimental Example 7. Animal experiments using HDAC6 inhibitor

### 7-1. Measurement of cancer cell volume and weight, cellular changes, and cytokine expression levels

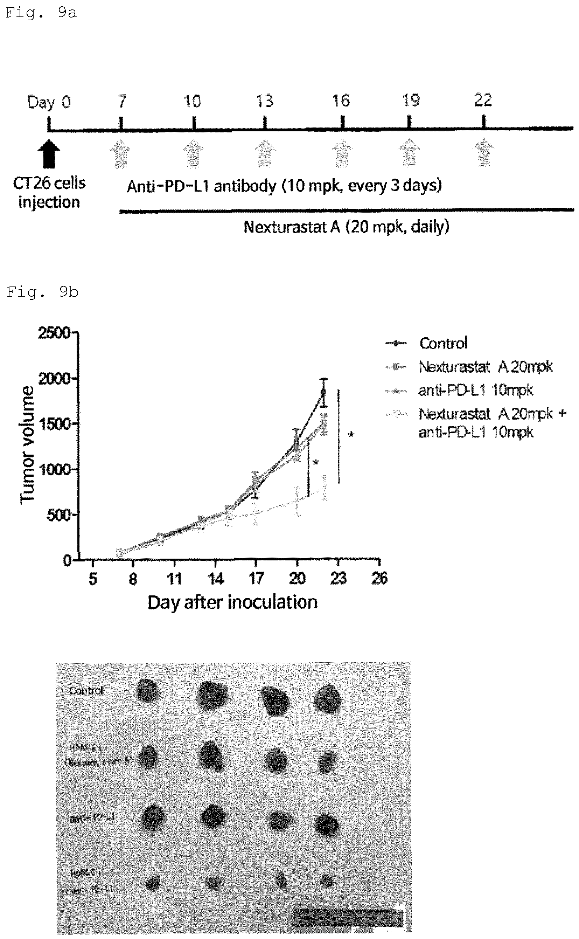

The animal experiment using an HDAC6 inhibitor was conducted using a total of three groups, a control group (control), an HDAC6i (Next A) group, and an HDAC6i (Next A)+anti-PD-L1 group. Nexturastat A (SellekChem) was used as HDAC6i (HDAC6 inhibitor), and #BE0101(Bioxcell) was used as an anti-PD-L1 antibody. CT26 (1x10⁶) cells and BALB/C mice were used. Three mice for the control group, three mice for the HDAC6i group, and four mice for the HDAC6i+anti-PD-L1 group were used. The treatment conditions were as follows, and are schematically shown in FIG. 9A.

### [Treatment conditions]

- HDAC6i group: HDAC6i 20 mpk(mg/kg) intraperitoneally injected daily
- HDAC6i+anti-PD-L1 group: HDAC6i 20 mpk intra peritoneally injected every day and anti-PD-L1 antibody 10 mpk intra peritoneally injected three times per week.

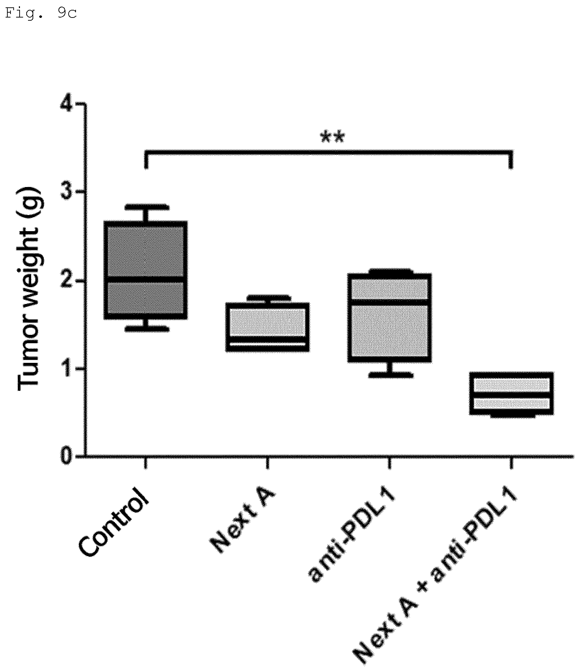

The tumor volume and weight were measured during the treatment with the HDAC6 inhibitor in the animal experiment, and the results are shown in FIGS. 9B and 9C. As a result, as can be confirmed from FIGS. 9B and 9C, the tumor volume and weight were further reduced in the treatment with the HDAC6 inhibitor and the ant-PD-L1 antibody in combination compared with the treatment with the HDAC6 inhibitor alone.

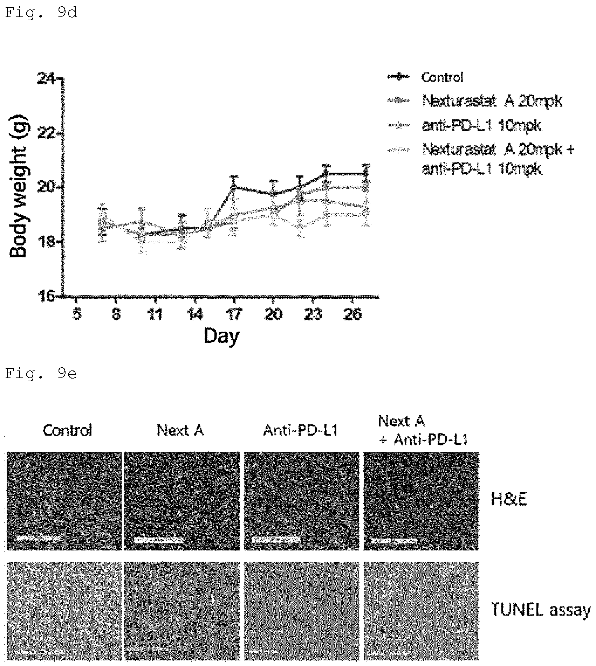

In the animal experiment, the body weight of the mice was measured during the treatment with HDAC6 inhibitor, and the results are shown in FIG. 9D, indicating that the body weight of the animals was not reduced by the drug.

In addition, paraffin blocks of tumor tissues were prepared for histological staining, and subjected to H&E and TUNEL assay in order to investigate cell death. The results are shown in FIG. 9E. As a result, as can be confirmed from FIG. 9E, the dead cells were increased by the treatment with the HDAC6 inhibitor and anti-PD-L1 antibody in combination compared with the treatment with the HDAC6 inhibitor alone.

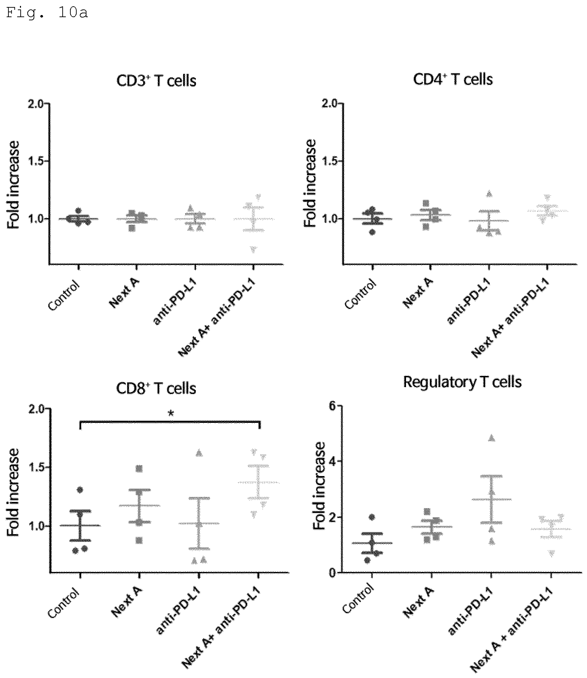

In addition, in the animal experiment according to the example of the present invention, flow cytometry (FACS) was used to measure the changes in CD3+ T cells, CD4+ T cells, CD8+ T cells, regulatory T cells, M1 macrophages, and M2 macrophages during the treatment with the HDAC6 inhibitor, anti-PD-L1 antibody, and HDAC6 inhibitor and anti-PD-L1 antibody in combination, and the results are shown in FIGS. 10A to 10C.

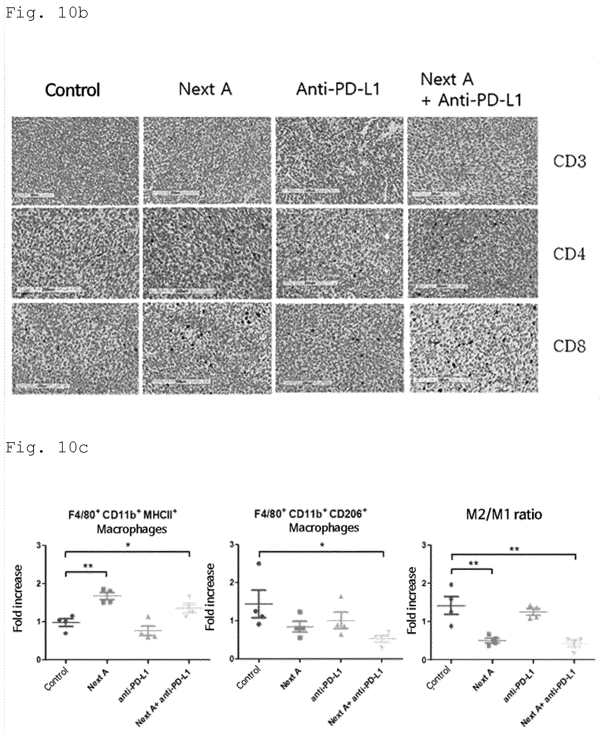

As can be confirmed from FIG. 10A, as a result of flow cytometry, the CD8+ T cells were increased by the treatment with the HDAC6 inhibitor and anti-PD-L1 antibody in combination, and as can be confirmed from FIG. 10B, histological staining confirmed an increase in CD8+ T cells. As can be confirmed from FIG. 10C, the treatment with the HDAC6 inhibitor and anti-PD-L1 antibody in combination showed an increase in M1 macrophages (MHCII⁺ macrophages) and a reduction in M2 macrophages (CD206⁺ macrophages), resulting in a decrease in M1/M2 ratio.

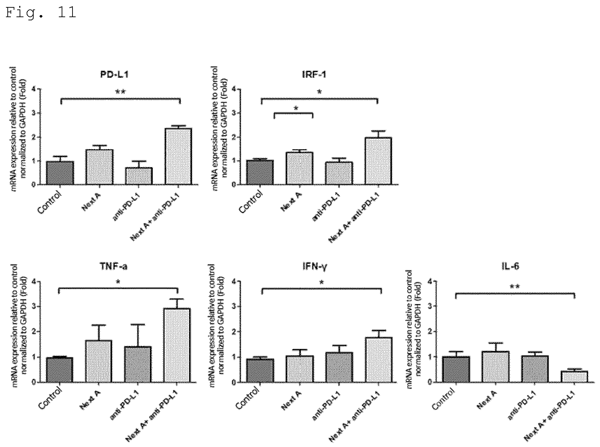

Additionally, the expression of related genes was examined by real-time PCR after the isolation of RNA form tumors. In the animal experiment according to an example of the present invention, the mRNA changes of PD-L1, TNF-a and IFN-γ (pro-inflammatory cytokines), IL-6 (anti-inflammatory cytokine), and IRF-1 (target gene for JAK2/STAT1 signaling) were measured, and the results are shown in FIG. 11. As a result, as can be confirmed from FIG. 11, the treatment with the HDAC6 inhibitor and anti-PD-L1 antibody in combination showed increases in expression of PD-L1 and TNF-a and IFN-γ (pro-inflammatory cytokines), a reduction in expression of IL-6 (anti-inflammatory cytokine), and an increase in expression of IRF-1 (target gene for JAK2/STAT1 signaling).

### 7-2. CD8 T cell-dependent tumor growth suppression

To examine the hypothesis that an HDAC6 inhibitor suppresses CT26 tumor growth in a CD8 T cell-dependent manner, an experiment on the combination with anti-CD8a antibody was further conducted by pretreatment with anti-CD8a antibody, which binds to CD8 T cells. The experiment was conducted by intraperitoneally administering an anti-CD8a antibody to animals from 3 days before tumor cell injection at 3-day intervals to inhibit CD8 T cells and administering an HDAC6 inhibitor and an anti-PD-L1 antibody.

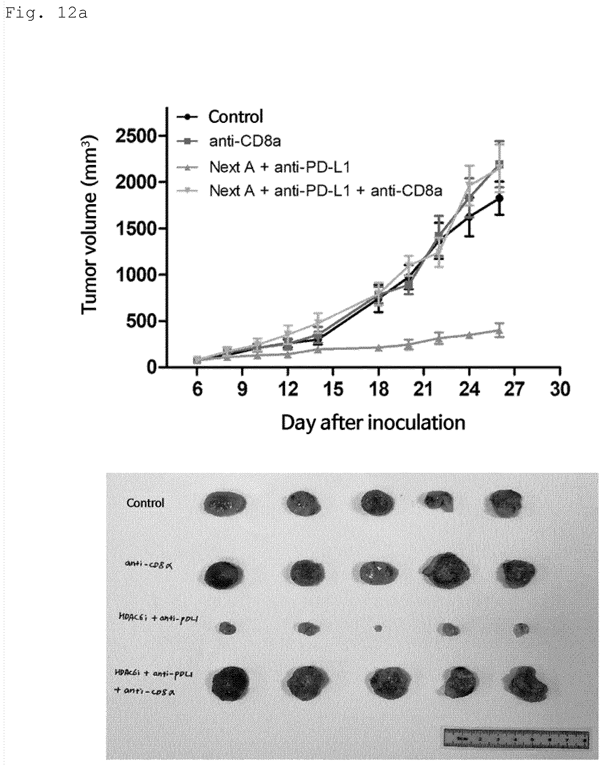

Specifically, the experiment was conducted on a total of four groups: a control group, an anti-CD8a group, an HDAC6i (Next A) + anti-PD-L1 group, and an HDAC6i (Next A) + anti-PD-L1 + anti-CD8a group. CT26 cells (1x10⁶) for a cell line, Nexturastat A (SellekChem) as HDAC6i (HDAC6 inhibitor), #BE0101 (Bioxcell) as an anti-PD-L1 antibody, and #BE017 (Bioxcell) as an anti-CD8a antibody were used. BALB/C mice were used. In addition, 6 mice for the control group, 6 mice for the anti-CD8a group, 6 mice for the HDAC6i (Next A) + anti-PD-L1 group, and 8 mice for the HDAC6i (Next A) + anti-PD-L1 + anti-CD8a group were used. The treatment conditions were as follows, and the experimental results are shown in FIGS. 12A to 12C.

### [Treatment conditions]

- anti-CD8a group: anti-CD8a antibody 10 mpk intraperitoneally injected to animals at 3-day intervals from 3 days before tumor cell injection.
- HDAC6i + anti-PD-L1 group: HDAC6i 20 mpk intra peritoneally injected to animals every day and PD-L1 antibody 10 mpk intraperitoneally injected three times per week, after tumor cell injection.
- HDAC6i + anti-PD-L1 + anti-CD8a group: anti-CD8a antibody 10 mpk intraperitoneally injected to animals at 3-day intervals from 3 days before tumor cell injection, and HDAC6i 20 mpk intraperitoneally injected to animals every day and PD-L1 antibody 10 mpk intraperitoneally injected three times per week, after tumor cell injection.

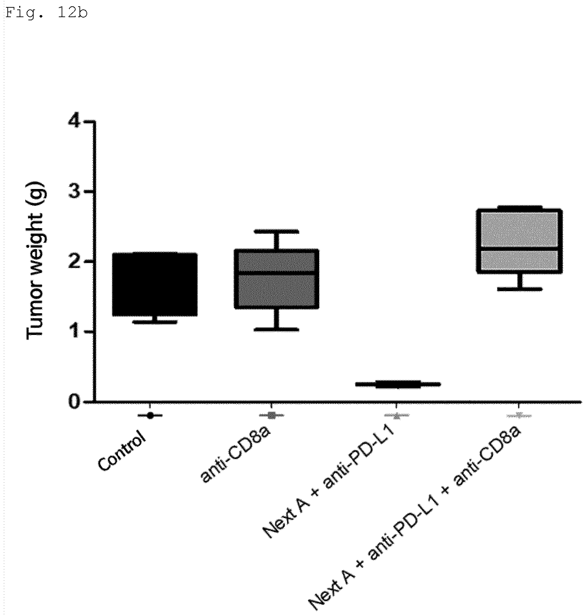

As a result, as can be confirmed from FIGS. 12A and 12B, upon the blocking of the CD8 T cells by anti-CD8 antibody, the CT26 tumor growth suppression and the tumor weight reduction were not observed by a combination of the HDAC6 inhibitor and anti-PD-L1 antibody, verifying the hypothesis that an HDAC6 inhibitor suppressed CT26 tumor growth in a CD8 T cell-dependent manner.

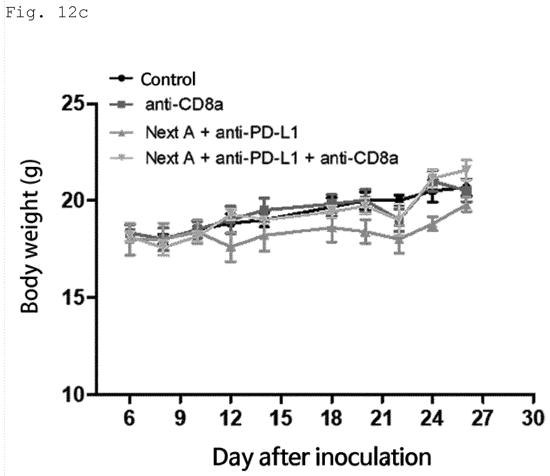

Additionally, the body weight of the mice was measured and is shown in FIG. 12, confirming that the body weight of the animals was not reduced by the drug.

### Experimental Example 8. Animal experiment using HDAC6 inhibitor and HDAC8 inhibitor

The animal experiment using an HDAC6 inhibitor and an HDAC8 inhibitor was conducted on a total of three groups: a control group, an HDAC6+8i group, and an HDAC6+8i + anti-PD-L1 group. Nexturastat A (SellekChem) as HDAC6i (HDAC6 inhibitor), PCI-34051 (SellekChem) as HDAC8i (HDAC8 inhibitor), and #BE0101(Bioxcell) as an anti-PD-L1 antibody were used. CT26 cell line (3x10⁶ cells) and BALB/C mice were used. Additionally, 3 mice for the control group, 3 mice for the HDAC6+8i group, and 4 mice for the HDAC6+8i + anti-PD-L1 group were used.

### [Treatment conditions]

- HDAC6+8i group: HDAC6i and HDAC8i each 25 mpk intraperitoneally injected three times per week.
- HDAC6+8i + anti-PD-L1 group: HDAC6i and HDAC8i each 25 mpk intraperitoneally injected three times per week and anti-PD-L1 antibody 10 mpk intraperitoneally injected two times per week -> PD-L1 antibody treatment the next day after HDACi treatment (HDACi - Monday, Thursday, Saturday/ anti-PD-L1 - Tuesday, Friday)

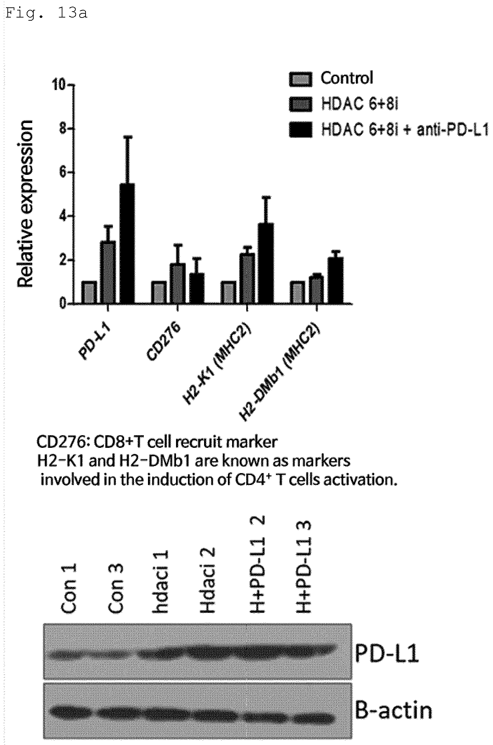

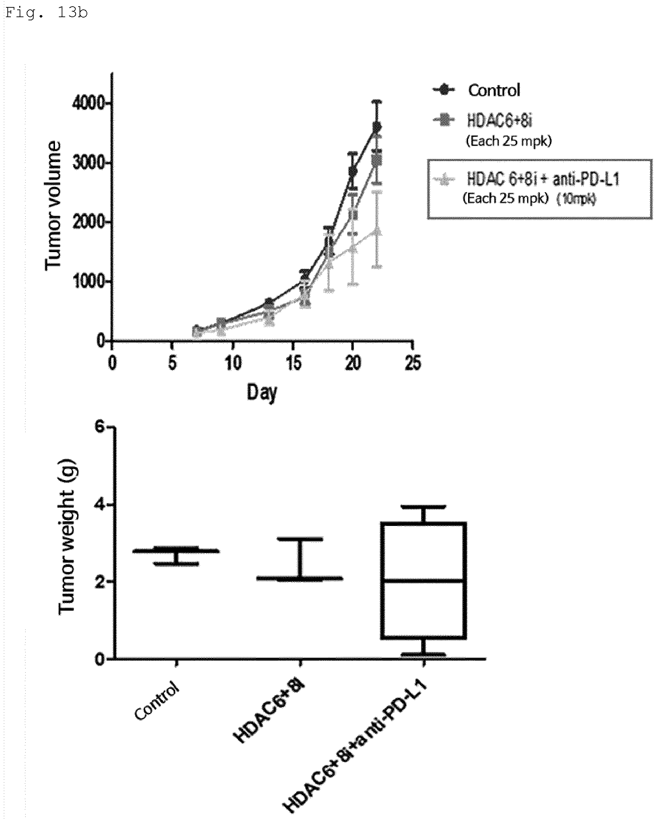

After the completion of the animal experiment, two mice were selected from each group considering the extent of tumor necrosis and size, and it was investigated whether the immune-related genes were up-regulated as hypothesized. The results are shown in FIGS. 13A and 13B.

As can be confirmed from FIG. 13A, the expression of PD-L1 was increased at the RNA and protein levels in the group treated with the HDAC6 inhibitor and the HDAC8 inhibitor. Additionally, the other immune-related genes (CD276, H2-K1, and H2-DMb1) as well as PD-L1 were up-regulated at the RNA level compared with the control group, and the expression of PD-L1 was also increased at the protein level.

As can be confirmed from FIG. 13B, the treatment with the HDAC6 inhibitor and the HDAC8 inhibitor in combination with the anti-PD-L1 antibody showed a smaller tumor size compared with the treatment with only the HDAC6 and HDAC8 inhibitors.

### Experimental Example 9. Comparative experiment between MSI and MSS colorectal cancer cell lines

### 9-1. Comparison of basal PD-L1 expression

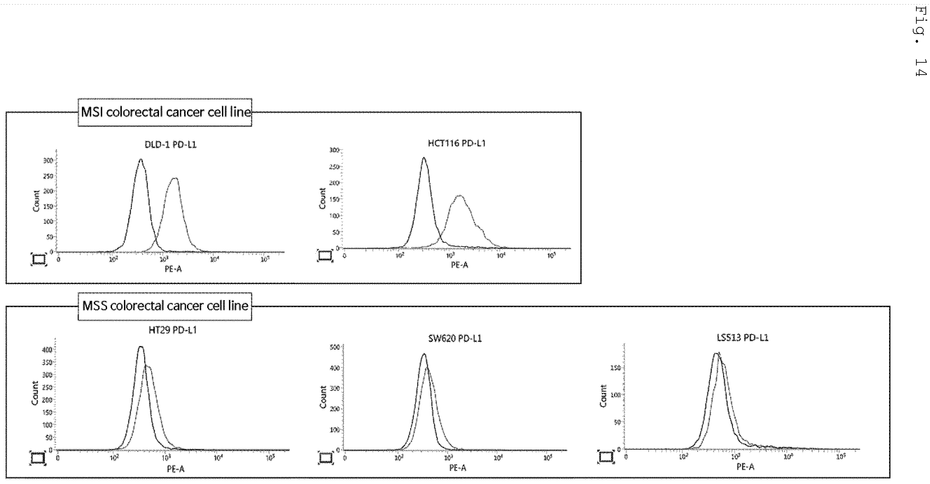

The basal PD-L1 expression levels in MSI and MSS colorectal cancer cell lines were compared through fluorescence-activated cell sorting (FACS), and the results are shown in FIG. 14. In the graphs of FIG. 14, the further the light gray solid line shifts to the right, the higher the expression level. The MSI colorectal cancer cell line showed a high expression of basal PD-L1, while the MSS colorectal cancer cell line showed a low expression of basal PD-L1.

PD-L1 (CD274), expressed on the surface of cancer cells, is an immune checkpoint protein and is involved in a mechanism where immune evasion is made by binding to PD-1 on the surface of T cells, and anti-PD-L1 antibodies can bind to PD-L1 on cancer cells to induce T cells to attack cancer cells, and thus in order to use anti-PD-L1 antibodies, PD-L1 needs to be expressed on the cancer cells. The results in FIG. 14 suggest that the use of the anti-PD-L1 antibody alone did not show a significant effect in the MSS colorectal cancer cell line due to a low expression of basal PD-L1.

It was therefore determined that the use of the pharmaceutical composition containing at least one selected from the group consisting of an HDAC6 inhibitor and an HDAC8 inhibitor in combination with an anti-PD-L1 antibody can exhibit a more effective cancer treatment in the MSS colorectal cell line.

### 9-2. Comparison between PD-L1 and B7H3 expressions by the treatment with HDAC6 inhibitor

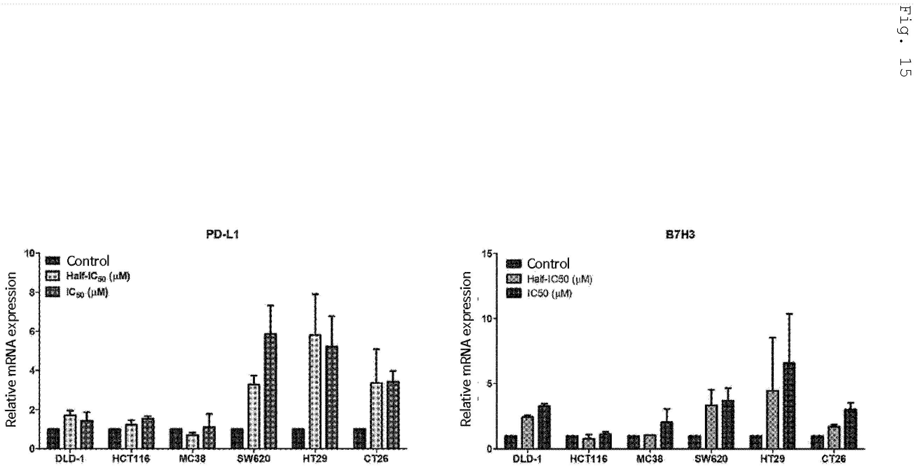

The IC₅₀ values of the HDAC6 inhibitor (Nexturastat A) in three MSI colorectal cancer cell lines (DLD-1, HCT116, and MC38) and three MSS colorectal cancer cell lines (SW620, HT29, and CT26) were determined. The cells were then treated with the inhibitor at the IC₅₀ and half-IC₅₀ concentrations (concentration corresponding to 1/2 of the IC₅₀ value), and the expression levels of the immune checkpoint proteins PD-L1 and B7H3 were measured. The results are shown in FIG. 15.

As shown in FIG. 15, distinct increase tendencies in PD-L1 and B7H3 expressions due to HDAC6 inhibition were observed in the MSS cell line compared with the MSI cell line. It was therefore determined that the use of an HDAC6 inhibitor can exhibit a more effective cancer treatment in the MSS colorectal cell line.

### Sub-conclusion

The reduction/suppression of HDAC6 and HDAC8 alone and the reduction/suppression of both HDAC6 and HDAC8 increased the expression of PD-L1 in the MSS colorectal cell lines, and the use thereof in combination with an anti-PD-L1 antibody exhibited a further enhanced tumor growth inhibitory effect. Hence, the pharmaceutical composition containing at least one selected from the group consisting of an HDAC6 inhibitor and an HDAC8 inhibitor according to the present invention is expected to be usable as an effective composition for cancer treatment.

### Industrial Applicability

The present invention relates to: a pharmaceutical composition for cancer treatment, an anticancer adjuvant for cancer immunotherapy, and a composition for increasing PD-L1 expression in cancer cells, each comprising at least one selected from the group consisting of a histone deacetylase (hereinafter, HDAC) 6 inhibitor and an HDAC8 inhibitor; a cancer treatment method and a method for increasing PD-L1 expression in cancer cells, each using at least one selected from the group consisting of an HDAC 6 inhibitor and an HDAC8 inhibitor; and uses of at least one selected from the group consisting of an HDAC 6 inhibitor and an HDAC8 inhibitor for cancer treatment, for anticancer adjuvant application in cancer immunotherapy, and for increasing PD-L1 expression in cancer cells.

## Claims

1. A pharmaceutical composition for cancer treatment, comprising at least one selected from the group consisting of a histone deacetylase (hereinafter, HDAC) 6 inhibitor and an HDAC8 inhibitor.

2. The pharmaceutical composition of claim 1, further comprising an anti-PD-L1 antibody.

3. The pharmaceutical composition of claim 1, wherein the cancer is at least one selected from the group consisting of colorectal cancer, pancreatic cancer, gastric cancer, and lung cancer.

4. An anticancer adjuvant for cancer immunotherapy, comprising at least one selected from the group consisting of a histone deacetylase (hereinafter, HDAC) 6 inhibitor and an HDAC8 inhibitor.

5. The anticancer adjuvant of claim 4, further comprising an anti-PD-L1 antibody.

6. The anticancer adjuvant of claim 4, wherein the cancer is at least one selected from the group consisting of colorectal cancer, pancreatic cancer, gastric cancer, and lung cancer.

7. A composition for increasing PD-L1 expression in cancer cells, the composition comprising at least one selected from the group consisting of a histone deacetylase (hereinafter, HDAC) 6 inhibitor and an HDAC8 inhibitor.

8. The composition of claim 7, wherein the cancer is at least one selected from the group consisting of colorectal cancer, pancreatic cancer, gastric cancer, and lung cancer.

9. A method for increasing PD-L1 expression in cancer cells, the method comprising bringing into contact with a subject at least one selected from the group consisting of a histone deacetylase (hereinafter, HDAC) 6 inhibitor and an HDAC8 inhibitor.

10. The method of claim 9, the cancer is at least one selected from the group consisting of colorectal cancer, pancreatic cancer, gastric cancer, and lung cancer.
